(19) Europäisches Patentamt
European Patent Office
Office européen des brevets

(11) Publication number: **0 186 347 B1**

(12) # EUROPEAN PATENT SPECIFICATION

(45) Date of publication of patent specification: **06.03.91**

(51) Int. Cl.5: **C12N 11/08**, C12N 11/14, C07K 17/08

(21) Application number: **85308832.6**

(22) Date of filing: **04.12.85**

(54) **Method for producing high-active biologically efficient compounds immobilized on a carrier.**

(30) Priority: **11.12.84 CS 9621/84**

(43) Date of publication of application:
**02.07.86 Bulletin 86/27**

(45) Publication of the grant of the patent:
**06.03.91 Bulletin 91/10**

(84) Designated Contracting States:
**AT BE CH DE FR GB IT LI LU NL SE**

(56) References cited:
**EP-A- 0 061 530**
**EP-A- 0 110 281**
**US-A- 4 451 568**

**BIOTECHNOLOGY AND BIOENGINEERING, vol. XXVI, 1984, pages 412-418, John Wiley & Sons, Inc.; H. TSUGE et al.: "Immobilization of yeast pyridoxaminephosphate oxidase to halogenoacetyl polysaccharides"**

(73) Proprietor: **TESSEK SDRUZENI PRAHA**
**Krizovnická 3**
**CS-110 00 Praha 1(CS)**

(72) Inventor: **Hermann, Peter**
**Mozartstrasse 21**
**Halle/S.(DE)**
Inventor: **Smalla, Kornelia**
**Ringfurtherweg 19**
**Magdeburg(DE)**
Inventor: **Turkova, Jaroslava**
**Julia Fucika 820**
**Cesky Brod.(CS)**
Inventor: **Coupek, Jiri**
**1580 Kotorska**
**Praha 4(CS)**
Inventor: **Wullhardt, Ingo**
**Block 762/03**
**Halle-Neustadt(CS)**

**EP 0 186 347 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

CHEMICAL ABSTRACTS, vol. 86, no. 22, 30th May 1977, page 198, abstract no. 166995d, Columbus, Ohio, US; Y. LIAN-WAN et al.: "Studies on immobilized enzyme III. Factors increasing the activity of immobilized glucoamylase"

BIOTECHNOLOGY AND BIOENGINEERING, vol. 24, no. 4, April 1982, page 837-845, John Wiley & Sons, Inc., New York, US; J. KOVAR et al.: "Immobilization of horse liver alcohol dehydrogenase on copolymers of glycidyl methacrylate and ethylene dimethacrylate"

JOURNAL OF CHROMATOGRAPHY, vol. 215, 1981, pages 165-179, Elsevier Scientific Publishing CO., Amsterdam, NL; J. TURKOVA et al.: "Hydroxaalkyl methacrylate gels derivatized with epichlorohydrin as supports for large-scale and high-performance affinity chromatography"

BIOTECH.APPL.BIOCHEM. (1988) 10, pp. 21-31

(74) Representative: Matthews, Graham Farrah et al
BROOKES & MARTIN Incorporating MATTHEWS, HADDAN & CO. High Holborn House
52/54, High Holborn
London, WC1V 6SE(GB)

**Description**

The invention pertains to an universal method for producing high-active biologically active compounds by immobilization with a covalent linkage on macroporous polymeric carriers in the presence of higher concentrations of inorganic salts.

Research and application of immobilized biologically active compounds represents nowadays an extensive field comprising both development and application projects of considerable practical importance.

The preparation and application of insoluble enzymes by bonding a soluble enzyme to a carrier enables to use the catalyst system repeatedly in a batch or through-flow arrangement of reaction in which the insoluble enzyme has a role of the specific heterogeneous catalyst. Such arrangement of the catalytic enzymatic reaction facilitates the subsequent separation of the enzyme from substrate and reaction products and lowers the cost and enables to automate the process in the multiple or continuous performance.

The rate of enzymatically catalyzed reaction depends, in the interaction of a substrate with the covalently immobilized enzyme, also on the character of linkage enzyme-carrier, on physical and chemical properties of the carrier (the size and distribution of pores, hydrophilicity of surface, etc.). Hydrodynamic parameters of the system, the flow rate of substrate, temperature, and others also significantly influence the final yield of reaction. The detailed knowledge of the factors affecting enzymatically catalyzed reactions utilizing the immobilized enzymes was obtained also in investigation the processes in preparative scale (penicillinacylase) (I. CHIBATA, Immobilized Enzymes, J. Wiley and Sons, New York 1870).

Immobilized biologically active compounds have found a very broad application in the affinity chromatography. This method employs the capacity of numerous biologically efficient efficient compounds to form sorption complexes with other compounds, while the character of interaction is very specific and reversible. If one of the components of a sorption complex is bonded to a solid carrier, only such compounds are selectively adsorbed from solution under suitable conditions which are noted for the specific affinity to the bonded compound. The sorption complex may be easily separated from other components in solution by means of the carrier. Dissociation of the sorption complex and separation of the soluble component from the component which remains chemically bonded to the carrier occur by changing the conditions (pH, ionic strength, temperature, addition of competitive sorbates, and the like). This procedure finds its use in the isolation and purification of enzymes, enzyme inhibitors, antidotes, antigens, soluble proteins, etc. Immobilization of bioactive compounds is advantageously used in biochemical analysis in the application of radioactive-labelled compounds or in optical labelling.

Numerous materials are used as carriers of biologically active compounds, for example, porous glass, silica gel, activated carbon, cellulose and its derivatives, starch, agarose, crosslinked polydextrans, synthetic polymers and copolymers as polyacrylamide, polystyrene, polyamides, poly(maleic anhydride), polyacrylates, polymethacrylates, etc. Some types are unsuitable because they carry ionogenic functional groups, other are noted for their strong nonspecific sorption properties for proteins. Other types have insufficient mechanical, hydrolytical, microbial or thermal stability or unsuitable distribution of pore size. These features considerably narrow their region of application. Homogeneous hydrophilic carriers of polysaccharide type mostly must not dry out, which fact makes difficult their storage and transportation.

The immobilization of enzymes on hydroplulic carriers off polysaccharide type in the presence of inorganic salts such as $(NH_4)_2SO_4$ has been described [see e.g. Biotech. Bioeng. VOL.26, May 1984, pp.412-418

Most of the above mentioned shortcomings are overcome in carriers prepared by copolymerization of 2-hydroxyalkyl methacrylates with alkylene dimethacrylates, which are activated for the purpose of immobilization reaction by substitution on the hydroxyl group (J. Coupek, J. Turkova, O. Hubalkova, M. Krivakova; Czechoslovak Patent No. 167,530).

Further detailed studies concerning the mechanism of immobilization of biologically active compounds revealed that the covalent linkage has to be as stable as possible towards hydrolysis, which cannot be satisfied, for example, by activation with cyanogen bromide or by amide, sulfide, ester or other bonding groups. Immobilization utilizing reactive epoxide groups according to the following scheme proved most suitable:

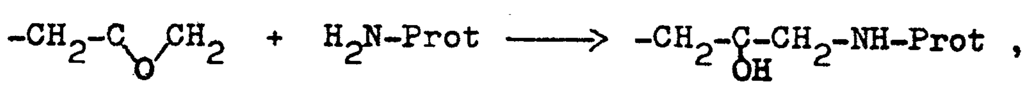

where Prot means the residue of protein.

Experiments with immobilization on the copolymer of glycidyl methacrylate with ethylene dimethacrylate (J. Turkova, K. Blaha, M. Malanikova, D. Vajenerova, F. Svec and J. Kalal; Biochem. Biophys. Acta 5424 (1978) 162) revealed that the reactive glycidyl methacrylate groups enclosed in a strongly crosslinked matrix of copolymer cannot be wholly employed for immobilization and that their subsequent deactivation is difficult. Therefore, a macroparous copolymer of 2-hydroxyethyl methacrylate with ethylene dimethacrylate was advantageously used as a carrier which was activated with epichlorohydrine and contains epoxypropyl functional groups only on the inner surface of porous ( J. Turkova, K. Blaha, J. Horacek, J. Vajener, A. Frydrychova and J. Coupek; J. Chromatogr. 215 (1981) 165-179) and on the surface of particles.

However, the yield of immobilized activity was rather low in some cases of direct bonding of protein on the epoxypropyl derivative of the hydroxyethyl methacrylate copolymer. This shortcoming, which occurs also in several other known activations of synthetic polymeric carriers, is removed by the subject of this invention.

According to the invention there is provided a method for producing highly active immobilized biologically active compounds useful for chemical transformation of compounds by enzymatic catalysis and for preparative and analytical separation techniques, by means of a hydrolytically stable covalent linking of said biologically active compounds to macro-porous polymeric carriers selected from the group comprising copolymers of hydroxyalkyl methacrylates ($C_2$ to $C_6$ -alkyl) with alkylene dimethacrylate ($C_2$ to $C_4$ -alkylene), copolymers of styrene with divinylbenzene or alkylene dimethacrylate ($C_2$ to $C_4$ -alkylene), copolymers of glycydil methacrylate with alkylene dimethacrylate. ($C_2$ to $C_4$ -alkylene), porous glass, and silica gel, said method comprising linking said biologically active compounds to the activated macroporous polymeric carrier in the presence of solutions of inorganic salts added in a concentration from 1.25 to 3 mol/l, the given concentration of inorganic salts present in the bonding reaction causing a two-step reaction, whereby a hydrophobic adsorption of the biologically active compound on carrier occurs in the first step and then, in the second step, the biologically active compound reacts with functional groups of the carrier, giving rise to a covalent linkage.

The said concentration of inorganic salts in the bonding reaction causes reduction or elimination of a solvation envelope of protein molecules.

The macroporous polymeric carriers are selected from the groups comprising synthetic copolymers of hydroxyalkyl methacrylates ($C_2$ to $C_6$ alkyl) with alkylene dimethacrylate ($C_2$ to $C_4$ alkylene), copolymers of styrene with divinylbenzene or alkylene dimethacrylate ($C_2$ to $C_4$ alkylene), copolymers of glycidyl methacrylate with ethylene dimethacrylate, porous glass, and silica gel.

The polymeric carrier has to be activated prior to the immobilization reaction. Reactive functional groups are selected from the group, comprising epoxides, aldehydes primary or secondary amines, carboxyls and thiols. The macroporous polymeric carrier has advantageously a rigid structure and sperical shape. To confirm the effect of the invention, they were chosen the compounds comprised in the group of enzymes, enzyme inhibitors, and also immunoactive proteins.

Salts, the presence of which in bonding of bioactive compounds substantially increases the yield of bonded protein and the yield of activity, are selected from the group comprising ammonium and sodium sulfates and phosphates. Reaction temperature is given by the reactivity of functional groups of the carrier and the bioactive compound determined for immobilization and is in the interval -5 °C to 30 °C. The reaction proceeds at pH 3-10 for 0.5 to 40 hours.

The method for immobilization of biologically active compounds in the presence of higher concentrations of inorganic salts according to the invention is highly universal and its mechanism was studied in details and confirmed with numerous biologically active compounds of various nature. The subject of invention is further elucidated and substantiated in several examples, which, however, do not limit its scope by any means.

Example 1

Enzyme aminoacylase (3.5.1.14)(10 mg) was dissolved in 5 ml of 0.2 M solution of phosphate buffer (pH 7). Ammonium sulphate was added, after the dissolution of protein had been completed, in such a way, that the final concentration of salt amounted to 1.75 mol/l of solution. Then, 100 mg of copolymer of 2-hydroxyethyl, methacrylate with ethylene dimethacrylate (SEPARON® 1000; exclusion limit $E_{max}$ = $2x10^6$ Dalton, particle size 40-80 µm, capacity of surface-bonded epoxide groups 1.77 mmol/g) was added. To speed up penetration of the solution into the macroporous structure of carrier, the suspension was kept under vacuum of a water-jet pump (15 mm Hg column) for 5 minutes. Immobilization of acylase was carried

out for 30 hours at 4 °C under moderate shaking of the suspension. After completed immobilization, the carrier was washed with a buffer solution (phosphate, pH 7) and with 1 M NaC1 solution. The content of bonded protein and its enzymatic activity were then determined. The yield of bonded activity was 10% with respect to the protein used and 20% with respect to the bonded protein.

Example 2

The reaction was carried out in the similar way as in example 1, with the distinction that it proceeded in the presence of an optimum amount of ammonium monohydrogensulfate (1.5 mol/1). The yields of activity were the same as in Example 1.

Example 3

Enzyme thermitase (3.4.21.14) (1 mg) was dissolved in 5 ml of 0.2 M phosphate buffer with pH 8.0. A solution of ammonium sulfate was added in such a way that the total concentration of salt amounted to 2.0 mol/1. Similarly as in example 1, 100 mg of a carrier was added, which contained epoxide groups (1.77 mmol/g) on the copolymer of 2-hydroxyethyl methacrylate with ethylene dimethacrylate, and the suspension was subjected to vacuum of a water-jet pump for 5 minutes. The reaction took 40 hours at 20 °C under moderate shking in this case. The yield of activity was 10%.

Example 4

The reaction was carried out analogously to example 3, with the distinction that an optimum concentration of added salt (sodium sulfate) was 1.25 mol/1. The reaction conditions and the yield of activity of the immobilized enzyme were the same as in example 3.

Example 5

To 1 mg of enzyme penicillinacylase (3.5.1.11.) in 5 ml of phosphate buffer (0.2 M, pH 8), it was added 100 mg of an activate carrier with particle size 100-200µm and then stepwise a solution of ammonium sulfate in such a way, that the final concentration of salt amounted to 2.5 mol/1. The reaction was carried out at 40 °C for 40 hours under moderate shaking. The reaction mixture was worked out in the same way as in example 1. The yield of activity was 45%.

Example 6

To 5 mg of enzyme elastase (3.4.21. 36.) in 5 ml of 0.2 M phosphate buffer of pH 8.0, it was added 100 mg of a copolymer of 2-hydroxypropyl methacrylate crosslinked with butylene dimethacrylate (exclusion limit $8x10^5$ Dalton; particle size 80-100µm) activated with epoxy groups (capacity 1.25 mmol/g). A solution of sodium hydrogensulfate was added up to the final concentration of 1.5 mol/1. The reaction took 20 hours at 4 °C under moderate shaking. The yield of activity was 17%.

Example 7

Cystathion- -synthetase (4.2.2.22.) (10 mg) was dissolved in 5 ml of 0.2 M TRIS-HC1 buffer solution at pH 8.7, 100 mg of a carrier, having the same composition as in example 1, was added followed by the solution of ammonium sulfate up to its optimum concentration of 2.5 mol/1. The reaction took 40 hours at -4 °C. The reaction mixture was worked out according to example 1. The yield of activity was 35%.

Example 8

To 1 mg of carboxypeptidase A (3.4.17.1.) dissolved in 5 ml of 0.2 M phosphate buffer, it was added 100 mg of a carrier prepared by copolymerization of glycidyl methacrylate with ethylene dimethacrylate 1:2 (exclusion limit $1x10^6$ Dalton, particle size 100-200 $\mu$m). The bonding reaction was carried out in the presence of 2.0 mol/1 of ammonium sulfate at 4 °C for 20 hours. The product was worked out according to example 1 and the yield of activity related to the protein added was determined at 11.5%.

Example 9

Anti-cathepsin D-IgG (3 mg) was dissolved in 5 ml of 0.2 M phosphate buffer at pH 8.0 and bonded to 100 mg of carrier, which was the same as in example 1, in the presence of 1.5 mol/1 ammonium sulfate for 24 hours at 25 °C under moderate shaking. After the reaction had been completed, the carrier with the bonded protein was isolated by filtration, washed with the above mentioned buffer solution, packed into a column and tested for application in the affinity chromatography of Cathepsin D-IgG.

Example 10

The carrier according to example 1 (250 mg) was **allowed to react at 50°C with 1 g of** hexamethylenediamine in 5 ml of water under moderate stirring for 3 hours. The reaction mixture was washed with water. The wet sorbent after washing was dispersed in 2 ml 0f 5% aqueous solution of glutaraldehyde, shaken for 20 hours and washed with water until the reaction with diphenylhydrazine was negative. The wet activated carrier was mixed with a solution of trypsin-inhibitor isolated from potatoes (1 mg/ml), 2 ml of 1.25 $Na_2SO_4$ was added, the reaction mixture was moderately shaken for 5 hours at 25 °C and the carrier was then filtered and washed with water. The immobilized inhibitor was used for the affinity chromatography of trypsin.

Example 11

Chymotrypsin (3.4.21.1.) (10 mg) was dissolved in 5 ml of 0.2 M phosphate buffer solution of pH 8 and 100 mg of carrier based on the epoxypropyl derivative of silica gel (exclusion limit $5x10^5$ Dalton, capacity 0.5 mmol/g) was added to the mixture. Ammonium sulfate (2.5 mol/1) was added and the bonding reaction was carried out for 20 hours at 30 °C. After washing according to example 1, the yield of activity of the bonded chymotrypsin was determined as 8.5%.

Example 12

The experiment was carried out analogously as in example 11 with the distinction that porous glass with the mean pore diameter 500 A was used as the carrier. The carrier was activated by reaction with triethoxyepoxypropylsilane. The yield of activity of the bonded chymotrypsin was 9.7%.

Example 13

Trypsin (3.4.21.4.) was dissolved in 5 ml of 0.2 M phosphate buffer with pH 8 and 100 mg of carrier according to example 1 was added. The reaction took 20 hours at the concentration of ammonium sulfate 2.5 mol/l and ambient temperature.

Example 14

Pepsin (3.4.23.1.) (10 mg) was mixed in 5 ml of 0.1 M acetate buffer of pH 4.0 with 100 mg of carrier similarly as in example 1. The reaction proceeded for 24 hours at the optimum concentration of ammonium sulfate 1.25 mol/l and 25 °C. After washing according to example 1, the yield of immobilized activity was found 15%.

Example 15

To 10 mg of insulin in 5 ml of 0.05 M phosphate buffer with pH 8, it was added 100 g of epoxy-activated carrier according to example 1. The bonding reaction was carried out in the presence of 1.75 mol/l of sodium monohydrogenphosphate at 25 °C for 50 hours. The bonded insulin was used for the affinity chromatography of antidotes against insulin from an antiinsulin serum in 0.1 M sodium barbiturate with pH 8.8, which contained 3% of albumin. The antidotes were desorbed with 3 M aqueous HC1.

## Claims

1. Method for producing highly active immobilized biologically active compounds useful for chemical transformation of compounds by enzymatic catalysis and for preparative and analytical separation techniques, by means of a hydrolytically stable covalent linking of said biologically active compounds to macro-porous polymeric carriers selected from the group comprising copolymers of hydroxyalkyl methacrylates ($C_2$ to $C_6$ -alkyl) with alkylene dimethacrylate ($C_2$ to $C_4$ -alkylene), copolymers of styrene with divinylbenzene or alkylene dimethacrylate ($C_2$ to $C_4$ -alkylene), copolymers of glycydil methacrylate with alkylene dimethacrylate ($C_2$ to $C_4$ -alkylene), porous glass, and silica gel, said method comprising linking said biologically active compounds to the activated macroporous polymeric carrier in the presence of solutions of inorganic salts added in a concentration from 1.25 to 3 mol/l, the given concentration of inorganic salts present in the bonding reaction causing a two-step reaction, whereby a hydrophobic adsorption of the biologically active compound on carrier occurs in the first step and then, in the second step, the biologically active compound reacts with functional groups of the carrier, giving rise to a covalent linkage.

2. The method according to Claim 1, wherein the macroporous polymeric carriers contain functional groups selected from the group comprising epoxides, aldehydes, primary and secondary amines, carboxyls and thiols.

3. The method according to Claims 1 or 2, wherein the macroporous polymeric carriers have a rigid structure and spherical form.

4. The method according to Claim 1, wherein the covalently bonded biologically active compounds are selected from the group comprising enzymes, enzyme inhibitors and immunoactive proteins.

5. The method according to Claim 1, wherein the salts are selected from the group comprising sodium phosphates, ammonium phosphates, sodium sulfates and ammonium sulfates.

6. The method according to Claim 1, wherein the bonding reaction is carried out in the temperature region from -5° to 30° C, in the region of pH3 to 10, and for 0.5 to 50 hours.

## Revendications

1. Procédé pour la production de composés immobilisés, biologiquement fortement actifs, pouvant être utilisés pour la transformation chimique de composés par catalyse enzymatique et pour des techniques de séparation préparative et analytique, au moyen d'une liaison covalente hydrolytiquement stable desdits composés biologiquement actifs à des supports polymère macroporeux choisis dans le groupe comprenant les copolymères de méthacrylates d'hydroxyalkyle (alkyle en $C_2$ à $C_6$) et de diméthacrylate d'alkylène (alkylène en $C_2$ à $C_4$), les copolymères du styrène et du divinylbenzène ou des diméthacrylates d'alkylène (alkylène en $C_2$ à $C_4$), les copolymères de méthacrylate de glycidyle et de diméthacrylates d'alkylène (alkylène en $C_2$ à $C_4$), le verre poreux et le gel de silice, ce procédé consistant à fixer les composés biologiquement actifs au support polymère macroporeux activé, en présence de solutions de sels minéraux ajoutés à une concentration de 1,25 à 3 moles/l, cette concentration de sels minéraux présents dans la réaction de fixation créant une réaction en deux étapes, la première étape consistant en une adsorption hydrophobe du composé biologiquement actif par le support, la deuxième étape consistant ensuite à faire réagir le composé biologiquement actif

avec des groupes fonctionnels du support pour donner une liaison covalente.

2. Procédé selon la revendication 1, dans lequel les supports polymères macroporeux contiennent des groupes fonctionnels choisis dans l'ensemble comprenant les époxydes, les aldéhydes, les amines primaires et secondaires, les carboxyles et les thiols.

3. Procédé selon les revendications 1 ou 2, dans lequel les supports polymères macroporeux ont une structure rigide et une forme sphérique.

4. Procédé selon la revendication 1, dans lequel les composés biologiquement actifs fixés par liaison covalente sont choisis dans l'ensemble comprenant les enzymes, les innibiteurs enzymatiques et les protéines immunoactives.

5. Procédé selon la revendication 1, dans lequel les sels sont choisis dans l'ensemble comprenant les phosphates de sodium, les phosphates d'ammonium, les sulfates de sodium et les sulfates d'ammonium.

6. Procédé selon la revendication 1, dans lequel la réaction de fixation est mise en oeuvre à une température de -5 à 30 ° C, à pH 3 à 10 et pendant 0,5 à 50 heures.

**Ansprüche**

1. Verfahren zur Herstellung hochaktiver, immobilisierter, biologisch aktiver Verbindungen, die zur chemischen Umsetzung von Verbindungen durch enzymatische Katalyse und für präparative und analytische Trennmethoden verwendet werden können, mit Hilfe einer hydrolytisch stabilen kovalenten Anbindung der biologisch aktiven Verbindungen an makroporöse polymere Träger, die aus der Gruppe, die Copolymere von Hydroxyalkylmethacrylaten ($C_{2-6}$-Alkyl) mit Alkylendimehacrylat ($C_{2-4}$-Alkylen), Copolymere von Styrol mit Divinylbenzol oder Alkylendimethacrylat ($C_{2-4}$-Alkylen), Copolymere von Glycidylmethacrylat mit Alkylendimethacrylat ($C_{2-4}$-Alkylen), poröses Glas und Kieselgel umfaßt, ausgewählt sind, das die Anbindung der biologisch aktiven Verbindungen an den aktivierten makroparösen polymeren Träger in dar Anwesenheit von Lösungen anorganischer Salze, die in einem Konzentration von 1,25 bis 3 mol/l zugefügt wurden, umfaßt; die angegebene Konzentration der anorganischen Salze, die bei der Bindungsreaktion anwesend sind, verursacht eine Zweistufenreaktion, dabei läuft in der ersten Stufe eine hydrophobe Adsorption der biologisch aktiven Verbindung an Träger ab und anschließend, in der zweiten Stufe, reagiert die biologisch aktive Verbindung mit funktionellen Gruppen des Trägers, um eine kovalente Bindung zu erzeugen.

2. Verfahren nach Anspruch 1, wobei die makroporösen polymeren Träger funktionelle Gruppen enthalten, die auf der Gruppe, die Epoxide, Aldehyde, primäre und sekundäre Amine, Carboxyle und Thiole umfaßt, ausgewählt werden.

3. Verfahren nach den Ansprüchen 1 oder 2, wobei die makroporösen Polymeren Träger eine feste Struktur und sphärische Form besitzen.

4. Verfahren nach Anspruch 1, wobei die kovalent gebundenen biologisch aktiven Verbindungen aus der Gruppe, die Enzyme, Enzyminhibitoren und immunoaktive Proteine umfaßt, ausgewählt werden.

5. Verfahren nach Anspruch 1, wobei die Salze aus der Gruppe, die Natriumphosphate, Ammoniumphosphate, Natriumsulfate und Ammoniumsulfate umfaßt, ausgewählt werden.

6. Verfahren nach Anspruch 1, wobei die Bindungsreaktion in dem Temperaturbereich von -5 bis 30 ° C, in einem pHWert-Bereich von 3 bis 10 und in einem Zeitintervall von 0,5 bis 50 Stunden durchgeführt wird.